# DEMANDE DE BREVET EUROPEEN

(11) **EP 1 136 480 A1**
(43) Date de publication de la demande: **26.09.2001**
(21) Numéro de dépôt: 00870052.8
(22) Date de dépôt: 23.03.2000
(51) Int. Cl.: C07D 319/12

(54) **Procedé de purification d'esters cycliques**

(71) Demandeur: Brussels Biotech, 1180 Bruxelles (BE)
(72) Inventeur: Coszach, M. Philippe, 6180 Courcelles (BE); Van Gansberghe, M. Frédéric, 1170 Bruxelles (BE); Di Salvatore, Patricia, 6183 Trazegnies (BE); Bogaert Jean-Christophe R.A., 7812 Ligne (BE)
(74) Mandataire: Vandeberg, Marie-Paule L.G.

(57) **Abrégé**

Procédé de purification de l'ester cyclique dimérique d'acide lactique (ou glycolique) au départ d'un brut de lactide (ou de glycolide) comportant des impuretés, le procédé comprenant :
- une cristallisation extractive et contrôlée du brut de lactide, en milieu aqueux, en contrôlant la géométrie des cristaux formés et en provoquant une ségrégation de phases entre le lactide et les impuretés;
- une séparation de la suspension de cristaux ainsi obtenue, en une phase liquide pauvre en lactide et chargée des impuretés, et en un gâteau humide riche en cristaux de lactide;
- un séchage du gâteau humide ainsi obtenu; et
- une recristallisation en milieu fondu du lactide impur séché ainsi obtenu et une récupération du lactide purifié.

## Description

### Domaine de l'invention

La présente invention concerne un procédé de purification d'esters cycliques dimériques, en particulier de lactides ou de glycolides. Les esters cycliques de formule générale : 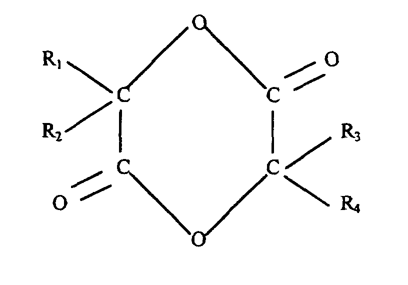 où R₁, R₂, R₃ et R₄ peuvent être soit un hydrogène, soit un groupement aromatique, soit un groupement aliphatique substitué ou non, ayant de 1 à 10 atomes de carbone, forment une classe très utile de composés pouvant être transformés en matière polymérique. De tels polymères sont particulièrement utiles pour la préparation de substances plastiques biodégradables dans l'environnement et se résorbent lorsqu'ils sont utilisés dans des applications médicales. Les polymères fabriqués au départ d'esters cycliques, tel que le lactide pour lequel R₁ = R₃ = H et R₂ = R₄ = CH₃, sont particulièrement appréciés car ils peuvent être dégradés au cours du temps par hydrolyse aqueuse dans la plupart des conditions environnementales. Les unités d'hydroxyacide (comme l'acide lactique) en résultant ou des oligomères, sont ensuite facilement absorbés par les micro-organismes dans l'environnement et ainsi convertis sous aérobiose en dioxyde de carbone et eau et, sous anaérobiose, en dioxyde de carbone et méthane. Les lactides sont également utiles en tant que plastifiants et intermédiaires pour la fabrication d'agents actifs de surface.

Il est bien connu qu'il existe deux formes optiquement actives d'acide lactique : acide L-lactique (L-LA) et acide D-lactique (D-LA). Dès lors, le lactide (LD), dimère cyclique de l'acide lactique, se présente sous trois formes diastéréoisomériques selon qu'il est constitué de deux molécules d'acide D-lactique (D,D-lactide ou D-LD), de deux molécules d'acide L-lactique (L,L-lactide ou L-LD), ou d'une molécule d'acide L-lactique et une molécule d'acide D-lactique (méso-lactide ou méso-LD). Signalons que l'on rencontre fréquemment un mélange racémique de D-lactide et de L-lactide appelé (D,L)-lactide et caractérisé par une température de fusion (Tf = 126°C) supérieure à celle des isomères L-LD et D-LD purs (Tf = 97°C).

Conformément aux pratiques antérieures, les lactides souhaités étaient synthétisés en effectuant une première condensation de l'acide lactique, en un prépolymère oligomérique d'un poids moléculaire relativement important. Ce dernier étant ensuite dépolymérisé à haute température et basse pression dans un réacteur chauffé afin de former un brut de lactide (brut de cyclisation), d'importants procédés de purification étaient ensuite nécessaires pour obtenir des lactides d'une pureté suffisante pour permettre la synthèse de polymères de poids moléculaire défini.

Actuellement, il existe deux grandes méthodes de production de lactide qui se distinguent essentiellement par le degré de polymérisation moyen (DP) des oligomères d'acide lactique produits lors de l'étape de condensation.

La première technique consiste à extraire l'eau d'une solution aqueuse d'acide lactique jusqu'à obtenir des oligomères d'acide lactique (8 ≤ DP ≤ 25). Ensuite, il convient de dépolymériser ces oligomères (réaction connue sous le nom de back-bitting) en présence d'un catalyseur acide de Lewis, soit sous pression réduite à température plus ou moins élevée, soit sous flux d'azote. Une autre variante concerne un procédé incluant d'abord la synthèse d'un prépolymère bloc incluant un noyau de polyéther thermiquement stable avec un hydroxyacide ou son ester polymérisé dans le noyau. Par chauffage sous vide ou flux gazeux, les extrémités de la chaîne d'hydroxyacide sont dégradées thermiquement pour former un ester cyclique pouvant être condensé sous vide. Ce procédé se réalise dans des conditions drastiques qui, d'une part, s'avèrent coûteuses et, d'autre part, influent sur la pureté optique du lactide (pourcentage élevé de méso-LD suite à des réactions de racémisation).

La seconde méthode utilise un oligomère de DP compris entre 1,5 et 2,5 pouvant être produit en phase vapeur à température élevée ou en phase liquide en présence d'un co-solvant formant un azéotrope avec l'eau. Cet oligomère peut être cyclisé en lactide dans des conditions d'autant moins drastiques qu'un solvant aromatique est utilisé pour extraire le lactide du milieu réactionnel. Les principaux inconvénients de ce procédé résident dans la présence d'un solvant souvent aromatique et de haut point d'ébullition, une température de réaction supérieure à 180°C, son manque de sélectivité et sa quantité non négligeable d'impuretés protiques (surtout carboxyliques).

De manière générale, le brut de lactide obtenu suite à ces diverses voies de synthèse contient toute une série d'impuretés protiques (acides carboxyliques, composés hydroxylés, eau, etc.) qu'il faudra extraire afin d'obtenir une pureté suffisante pour pouvoir intégrer le brut au procédé de polymérisation par ouverture de cycle.

### Etat de la technique

L'homme de l'art connaît le document US 5,264,592 qui au départ d'un brut de lactide, permet par un procédé de purification par cristallisation à l'état fondu réalisé de façon dynamique et en multi-étapes, d'obtenir une teneur en lactides supérieure à 99 % en poids. Les pourcentages sont toujours exprimés en poids dans la suite de ce texte.

Le procédé présente plusieurs inconvénients :
- il nécessite de partir d'un brut de lactide déjà très riche en lactides (>95%) avec des teneurs limitées en acide lactique avec ses oligomères et en eau;
- le méso-LD n'est pas considéré comme un produit commercialement valorisable, alors qu'il convient tout à fait pour la synthèse de certains polylactides facilement biodégradables;
- le procédé en continu proposé peut fournir une pureté raisonnable, mais au prix d'un faible rendement et d'un appareillage complexe.

Mais l'inconvénient majeur de cet enseignement est le fait qu'il ne concerne que des bruts de lactide présentant un rapport L-LD/méso-LD d'au moins 80/20, préférablement de 90/10. En effet, si la composition du brut de lactide se trouve correspondre à une composition au delà de l'eutectique, on assiste alors à un enrichissement en méso-LD, avec rejet du L-LD avec les impuretés comme l'eau, l'acide lactique et les oligomères.

D'autres documents comme CA 2 115 472, proposent également un procédé de purification par cristallisation dynamique à l'état fondu. On y envisage la récupération du méso-LD sous une forme enrichie. Mais le procédé n'est applicable qu'à des bruts de lactide présentant des rapports L-LD/D-LD d'au moins 80/20 ou d'au moins 20/80. En effet, si la composition du brut se trouve correspondre à une composition au-delà de l'eutectique, on assiste alors à un enrichissement en mélange racémique L-LD + D-LD, avec rejet du L-LD avec les impuretés comme l'eau, l'acide lactique et les oligomères. D'autre part, il est nécessaire de partir d'un brut de lactide déjà riche en lactides (>90% L-LD + méso-LD).
D'autres documents comme US 5,502,215 et JP 10025288 décrivent une purification au moyen d'une cristallisation en milieu aqueux, complétée notamment par une recristallisation en solvant organique et éventuellement un rinçage avec ce solvant organique.

US 5,502,215 concerne un procédé de purification de brut de lactide, comprenant une cristallisation en milieu aqueux de L-LD et/ou D-LD, puis une séparation centrifuge, un séchage en phase gazeuse et une recristallisation en solvant organique avec séparation centrifuge et séchage en phase gazeuse. L'accent est mis sur l'élimination par hydrolyse du méso-LD et non sur une extraction des impuretés protiques par l'eau. D'autre part, on ne cherche pas à produire de cristaux de lactide d'un type particulier.

JP 10025288 concerne une méthode de raffinage d'un mélange de lactides, comprenant une cristallisation en milieu aqueux de L-LD et/ou D-LD, une séparation puis un rinçage des cristaux par solvant organique non polaire, avant une séparation et un rinçage avec le même solvant non polaire puis séchage en phase gazeuse. L'accent est aussi mis sur l'élimination du méso-LD.

Les deux méthodes de purifications décrites ci-dessus, permettent de traiter divers bruts de lactide et d'obtenir des puretés de l'ordre de 99 %, autorisant une polymérisation en polylactide dans des conditions raisonnables. Cependant, ces méthodes de purification entraînent soit des pertes de rendement importantes dues à l'ouverture, la racémisation chimique et/ou thermique du cycle lactide, soit des investissements et des coûts d'exploitation importants suite aux nécessités de stockage et de traitement liées à une purification avec solvant.

La présente invention pallie ces inconvénients, tout en permettant de produire un lactide suffisamment pur en vue de la polymérisation dans de bonnes conditions économiques.

### Brève description de l'invention

La présente invention consiste en un procédé de purification d'esters cycliques, en particulier du dimère cyclique de l'acide lactique (le lactide) au départ d'un "brut de lactide" à savoir un mélange d'acide lactique et/ou d'ester d'acide lactique et de leurs oligomères respectifs (LₙA avec n < 5), d'eau et/ou d'alcool ainsi que des différentes formes diastéréoisomériques du lactide.

Ce brut de lactide peut être obtenu soit au départ d'acide lactique, et/ou de ses sels et/ou de ses esters provenant de toute voie de synthèse connue de l'homme de l'art et dont une description non exhaustive a été développée ci-avant, soit au départ de fractions constituées des résidus de procédés de purification comme par exemple la distillation ou la cristallisation en milieu fondu.

Dans la suite de la présente demande, toutes les descriptions feront référence à la synthèse de lactide au départ d'acide lactique. Cependant, elles pourront également s'appliquer aux esters d'acide lactique. D'autre part, par lactide on entend l'une des deux formes diastéréoisomériques à savoir soit le L-LD, soit le D-LD et non le méso-LD.

Le procédé de purification décrit dans cette invention est original dans le sens où il assure, au départ d'un brut de lactide (même à concentration relativement basse en lactide), l'obtention d'une très haute qualité de lactide avec un rendement massique élevé et une consommation énergétique minimale. On entend par lactide de très haute qualité, un lactide d'une pureté chimique et optique suffisante pour pouvoir être utilisé comme monomère pour la synthèse du polylactide (PLA) par ouverture de cycle.

Les aspects quantitatif et sélectif de ce procédé de synthèse sont avantageusement assurés par la mise en oeuvre conjointe : (a) d'une cristallisation extractive et contrôlée du lactide en milieu aqueux afin de favoriser la formation de gros cristaux ainsi que le transfert des impuretés protiques en phase liquide, (b) d'une séparation centrifuge ou autre (avec ou sans lavage) du lactide et de la phase aqueuse, (c) d'un séchage en phase solide ou liquide du gâteau humide obtenu et (d) d'une ou plusieurs recristallisations en milieu fondu.

Cette séquence permet un recyclage aisé et quantitatif des impuretés en phase aqueuse au sein du procédé de production d'acide lactique. De plus, l'optimisation des conditions de température et de temps de séjour de ce procédé permet, contrairement aux procédés conventionnels, d'éviter les détériorations chimiques et thermiques du lactide durant la phase de purification. Dès lors, les critères de qualité ainsi que de rendement indispensable pour toute exploitation industrielle sont atteints beaucoup plus facilement.

L'objectif énergétique minimal est quant à lui atteint par la simplicité des technologies mises en oeuvre, les faibles températures de travail ainsi que la judicieuse juxtaposition des étapes. En effet, la recristallisation en milieu fondu est connue de l'homme de l'art, comme une technologie permettant l'obtention d'un lactide d'une qualité irréprochable et une sélectivité requise pour la synthèse du PLA. Cependant, au départ d'un brut de lactide de relativement basse teneur en lactide, cette technologie ne peut à la fois garantir un rendement en lactide exploitable industriellement et soutenir une comparaison économique (ampleur des investissements) vis-à-vis des autres technologies plus conventionnelles (distillation, recristallisation en solvant, etc.). Par contre, l'approche est toute autre si la succession des étapes (a) à (d) ainsi que la méthodologie préconisée par la présente invention sont respectées.

### Description détaillée de l'invention

De manière préférée, le mélange de départ aura une composition relative en un des lactides comprise entre 30 et 90 % et préférablement entre 40 et 85 %, en eau (lors d'un travail avec un ester, l'eau sera remplacée par un alcool) comprise entre 0 et 2 % et préférablement entre 0 et 1 %, en acide lactique et ses oligomères (LₙA avec n < 5) comprise entre 0 et 50 %, le solde constitué par le méso-LD et l'autre diastéréoisomère du lactide, est compris entre 0 et 30 %.

Ce mélange ou brut de lactide provient de façon privilégiée de l'extraction à un moment précis de l'étape de condensation des vapeurs issues de la synthèse du dimère cyclique de l'acide lactique. Une seconde approche privilégiée de la présente invention consiste à récupérer les fractions issues de procédés de purification, tels que la distillation ou la cristallisation en milieu fondu, dont la teneur en lactide est trop faible pour être directement purifiées mais suffisante pour justifier un recyclage en tant que lactide et non comme source d'acide lactique, provenant de l'hydrolyse du lactide.

Ce procédé comprend essentiellement les étapes suivantes :

### (a) une cristallisation extractive et contrôlée.

Cette première étape consiste en une cristallisation, quantitative, sélective et contrôlée en milieu aqueux du lactide avec concentration des impuretés protiques en phase liquide, par ajout d'eau.

Cette première étape couplée à celle de la séparation centrifuge (b) et celle de séchage (c) constitue une prépurification destinée à fournir un mélange de sélectivité (teneur en LD) suffisante pour pouvoir être traité efficacement et de façon rentable lors de l'étape ultime de purification, à savoir la recristallisation en milieu fondu (d) . Par sélectivité élevée, on entend une teneur en LD supérieure à 90 % et préférablement supérieure à 95 %, sans tenir compte de l'eau ajoutée.

Le but de cette étape par rapport aux procédés d'extraction à l'eau déjà connus, n'est pas d'éliminer la majeure partie du méso-LD par hydrolyse du cycle, mais bien de contrôler la géométrie des cristaux formés, également de provoquer une ségrégation de phases entre le lactide (phase solide) et les impuretés (phase liquide), et enfin de favoriser l'extraction des impuretés protiques solubles dans l'eau. Il est évident qu'une diminution de la teneur en méso-LD ne pourra être évitée, mais elle n'est nullement recherchée. L'étape ultime du procédé permettant effectivement une séparation stéréospécifique du lactide et du méso-LD, il convient d'éviter lors des étapes précédentes d'hydrolyser ce dernier par ouverture de cycle. Pour certaines applications où le contrôle de la dégradation est utile, la récupération du méso-LD et son utilisation lors de la synthèse de certains PLA constituent un atout majeur.

L'avantage de ce procédé réside dans la grande facilité offerte au recyclage des impuretés vers la production de lactide à partir d'acide lactique, dans le contrôle de la géométrie des cristaux, dans l'efficacité de l'extraction aqueuse des impuretés protiques, et dans les conditions réactionnelles très douces qui permettent d'éviter les pertes de rendement occasionnées par une ouverture chimique ou thermique du lactide.

En effet, la présente invention préconise des températures initiales et finales du mélange [brut de lactide + eau servant à l'extraction] n'excédant pas respectivement 100°C et 50°C, préférablement inférieures à 90°C et 35°C et plus préférablement encore inférieures à 80°C et 25°C, et des temps de séjour compris entre 1 et 90 min, de préférence compris entre 1 et 60 min pour initier et compléter l'extraction, ce qui élimine tout problème de racémisation, réduit drastiquement les dépenses énergétiques et augmente la productivité du procédé.

D'autre part, l'ajout d'une quantité inadaptée d'eau peut créer des problèmes de transfert, réduire l'efficacité de l'extraction ou empêcher tout contrôle de la cristallisation. En effet, l'ajout d'une quantité trop importante d'eau permet une hydrolyse importante du méso-LD mais entraîne également une hydrolyse du lactide par ouverture de cycle, ce qui influe considérablement sur le rendement de l'étape. Dans les procédés d'extraction connus, cette dégradation a pu être freinée par une diminution très rapide de la température du mélange. Cependant cette brusque chute de température provoque une nucléation très importante au sein du mélange qui se traduit par sa prise en masse. Ceci n'est pas problématique pour les procédés d'extraction connus car leur principal objectif réside en une hydrolyse sélective du méso-LD présent sous forme cristalline et non en une extraction aqueuse des impuretés protiques, la pureté du produit de départ étant déjà relativement élevée. Par contre, en ce qui concerne notre approche, la nucléation ainsi que la croissance des cristaux doivent être contrôlées afin d'éviter cette prise en masse qui amoindrit drastiquement l'efficacité de l'extraction. En effet, cette chute de température provoque d'une part, une hausse de la viscosité des impuretés (tels l'acide lactique ou les oligomères d'acide lactique) qui s'élimineront beaucoup plus difficilement de la surface des cristaux et d'autre part, la formation d'un bloc qui sera plus difficilement solvaté par l'eau.

De plus, cette prise en masse empêchera tout contrôle de la géométrie des cristaux qui ne pourront développer une structure lamellaire. Ces cristaux sans inclusions et occlusions formés suivant l'invention, sont beaucoup plus purs, stables et faciles à manutentionner.

Dans le cadre de cette invention, notre souci n'étant pas de provoquer une hydrolyse du méso-LD, la concentration en eau ajoutée au mélange de départ sera moindre, à savoir comprise entre 0 et 40 %, préférablement comprise entre 0 et 30 % et plus préférablement comprise entre 0 et 20%. De ce fait, la dégradation du lactide est beaucoup plus lente et permet un meilleur contrôle de la température et de la cristallisation en cours de procédé. En effet, comme à chaque composition en lactide du brut de lactide correspond une température de cristallisation spécifique, dans la première phase du procédé, le mélange sera amené à 10°C, préférablement à 5°C et préférablement encore à 2°C en dessous de cette température et y sera maintenu entre 1 et 45 min, préférablement entre 1 et 30 min, et plus préférablement encore entre 1 et 15 min. Une approche de la présente invention consiste à fixer la température de l'eau ajoutée de sorte qu'une fois le mélange effectué, sa température corresponde à la température de maintien souhaitée. Une seconde approche de la présente invention consiste à initier la cristallisation par l'ajout dans la solution, de cristaux de lactide pur dans le but d'amorcer la germination progressive.

Durant la phase suivante de la première étape, la température du mélange est lentement diminuée de manière à engendrer la croissance progressive des cristaux et à augmenter le rendement du procédé. Par ce contrôle de la cristallisation, les impuretés sont progressivement repoussées en phase liquide et des cristaux à structure lamellaire sans inclusions sont formés. Cette méthode de travail est d'autant plus avantageuse que cette géométrie de cristaux augmente sensiblement l'efficacité des deux étapes suivantes du procédé à savoir la séparation (b) et le séchage (c). D'autre part, il a été observé que pour une teneur en eau voisine de 1 %, la stabilité chimique des cristaux de lactide est accrue vis-à-vis de ceux obtenus par prise en masse du mélange.

Dans la présente invention, la configuration du réacteur et de son agitation est importante afin de maintenir un contrôle de la cristallisation. Le réacteur sera avantageusement conçu pour pouvoir offrir d'une part, une bonne agitation afin d'assurer une répartition de la chaleur sur l'ensemble du mélange, d'éviter la prise en masse et de permettre l'évacuation du mélange hors du réacteur, et d'autre part, une capacité de thermostatisation importante afin de favoriser une cristallisation progressive (rendement) et contrôlée (croissance des cristaux) du lactide. Dès lors, dans le cadre de cette invention, tout réacteur par charge ou en continu, susceptible de remplir ces exigences peut être utilisé, comme par exemple un réacteur par charge couplé à un échangeur externe de chaleur. De plus, plusieurs technologies connues de l'homme de l'art peuvent également être envisagées, afin de favoriser la cristallisation du lactide, comme par exemple : la sonocristallisation, l'ensemencement par cristaux, etc.

Il est primordial de noter que contrairement aux procédés d'extraction à l'eau déjà connus, cette nouvelle méthode de travail favorise l'apparition en concentration plus ou moins importante d'une forme particulière de lactide.

En effet, lors de l'analyse des produits issus de cette étape de cristallisation extractive et contrôlée, nous avons été fort surpris d'observer dans nos analyses GC l'apparition d'un composé supplémentaire ne correspondant à aucun produit connu.

Une analyse plus approfondie des différentes données nous a permis de montrer qu'en fonction de l'évolution des températures, de la concentration en eau et du temps de contact, la teneur de ce composé évoluait de façon inverse à celle du lactide.

Des analyses complémentaires ont été réalisées par GC-MS, RMN (¹³C, ¹H) et IR, sur des échantillons contenant des teneurs variables de ce composé. Il est apparu que ce composé n'était autre qu'une molécule de lactide « complexée » par une molécule d'eau. Par « complexée », nous entendons l'existence d'une interaction polaire relativement forte mais pas d'un lien chimique covalent. En effet, l'analyse des deux molécules (le lactide et le complexe) en spectrométrie de masse a fourni deux spectres totalement identiques, sans pic supplémentaire à m/z 162, ce qui tend à prouver que nous sommes en présence d'un complexe et non d'un lien chimique. De plus, les spectres RMN et IR montrent bien une modification du complexe par rapport au lactide, modification correspondant à la présence d'eau.

Il est également important de noter que lors d'un refroidissement brusque similaire à ceux opérés dans les procédés connus de l'état de l'art, ce complexe n'est pas généré. Or la connaissance de l'existence et de la nature de ce complexe est avantageuse pour une gestion correcte et efficace des étapes de séchage et de cristallisation en milieu fondu liées à ce procédé.

### (b) une séparation centrifuge.

Cette étape de l'invention consiste au départ d'une suspension obtenue en (a) et caractérisée par une teneur en eau dans le mélange de départ comprise entre 1 et 40 % en poids, préférablement comprise entre 1 et 25 % et plus préférablement encore comprise entre 1 et 20 %, une teneur en un lactide (complexe inclus) comprise entre 35 et 90 %, préférablement entre 40 et 90 % et plus préférablement encore comprise entre 45 et 90 %, une teneur en acide lactique et ses oligomères (LₙA avec n<5) comprise entre 0 et 10 % et préférablement comprise entre 0 et 5 %, le solde étant constitué par le méso-LD et l'autre diastéréoisomère du lactide, en une séparation centrifuge ou autre du lactide, essentiellement présent dans la phase solide (gâteau), et des filtrats aqueux chargés en impuretés protiques.

Les filtrats générés à cette étape pourront être facilement recyclés au sein du procédé de production de lactide à partir d'acide lactique, ce qui permettra d'augmenter le rendement global du procédé visant la synthèse du PLA au départ de divers substrats carbonés.

Dans le cadre de cette invention, nous avons démontré qu'une séparation centrifuge est souhaitable, car très rapidement en raison de la géométrie favorable des cristaux générés dans l'étape (a), la siccité du gâteau étant suffisante pour permettre une manutention aisée du produit. D'autre part la stabilité chimique suffisante des cristaux évite toute perte de rendement par ouverture de cycle.

Une approche privilégiée de la présente invention considère un temps d'essorage suffisant, de façon à atteindre des teneurs en eau résiduelle libre comprises entre 0 et 3%, préférablement comprises entre 0 et 1% et plus préférablement comprises entre 0 et 0,5%.

Afin de faciliter ou de supprimer l'étape suivante de séchage, une approche particulière de la présente invention considère un lavage du gâteau d'essorage. Les avantages liés à ce lavage sont multiples. En effet, il permet de réduire les temps de contact au strict minimum ce qui est bénéfique pour le rendement. Tout procédé connu de l'homme de l'art susceptible de faciliter le lavage du gâteau d'essorage, conviendra dans le cadre de cette invention.

D'autre part, le choix judicieux du solvant de lavage permet par un moyen physique simple d'éliminer les impuretés déposées sous forme de film à la surface des cristaux, de réduire la teneur en eau résiduelle du gâteau et donc d'augmenter la stabilité chimique des cristaux. Idéalement, le solvant utilisé devrait notamment être miscible à l'eau (diminution de l'eau résiduelle), former un azéotrope inférieur avec l'eau (distillation plus aisée des traces d'eau), avoir un point d'ébullition relativement bas (économie), être inerte chimiquement vis-à-vis du lactide (éviter l'ouverture du cycle), avoir une solubilité faible vis-à-vis du lactide (éviter les pertes de rendement), avoir une interaction avec l'eau supérieure à celle du lactide (éliminer l'eau liée au lactide). Il sera difficile de sélectionner un solvant répondant à tous ces critères. Le choix du solvant résultera donc d'un compromis qui mettra en avant l'efficacité de l'extraction, le rendement ainsi que la rentabilité du procédé.

Parmi les solvants utilisables, mentionnons des cétones, des éthers, des hydrocarbures aromatiques ou aliphatiques, des solvants à base de silicone et des solvants halogénés, par exemple : acétone, 2-butanone, 2-pentanone, 2-hexanone, 2-heptanone, 2-octanone, anisole, éthyléther, isopropyléther, butyléther, méthylphényléther, méthylisobutylcétone, benzène, cumène, cymène, p-xylène, o-xylène, m-xylène, toluène, cyclohexane, hexane, heptane, octane, nonane, 1-pentène, 4-méthylanisole, 1,2-diméthoxybenzène, 1,3-diméthoxybenzène, 1,4-diméthoxybenzène, mésitylène, chlorobenzène, 1,2-dichlorobenzène, 1,3-dichlorobenzène, 1,4-dichlorobenzène, 2-chlorotoluène, 3-chlorotoluène, 4-chlorotoluène, éthanol, isopropanol.

### (c) le séchage .

Cette étape de l'invention consiste, au départ d'un gâteau humide obtenu en (b) et caractérisé par une teneur en eau comprise entre 0 et 5 %, préférablement comprise entre 0 et 2 % et plus préférablement encore comprise entre 0 et 1 %, une teneur en un lactide (complexe inclus) comprise entre 75 et 98 %, préférablement comprise entre 85 et 98 % et plus préférablement encore comprise entre 90 et 98 %, une teneur en acide lactique et ses oligomères (LₙA avec n<5) comprise entre 0 et 5 %, préférablement comprise entre 0 et 3 % et plus préférablement encore comprise entre 0 et 1 %, le solde étant constitué par le méso-LD et l'autre diastéréoisomère du lactide, en une évaporation des teneurs résiduelles d'eau. Si nécessaire, cette étape peut également être mise à profit pour extraire totalement ou partiellement le solvant introduit lors du lavage.

De plus, le complexe qui est généré dans le cadre de ce procédé devra être minutieusement géré de façon à assurer l'efficacité du procédé. En effet, nous avons remarqué que la formation de ce complexe était réversible sous certaines conditions et que le complexe pouvait donc relarguer son eau.

Dès lors, le gâteau humide issu de l'étape (b) devra être traité en considérant qu'il renferme une eau résiduelle libre, mais également une eau liée stockée sous forme de complexe.

La faible teneur en eau (libre +liée) du lactide humide, bien qu'utile pour assurer la stabilité chimique temporaire du lactide, ne permet pas une ultime purification efficace et rentable par recristallisation en milieu fondu. Une teneur en eau (libre + liée) trop importante est source d'une perte de rendement par ouverture de cycle lors des refontes. Une approche privilégiée de la présente invention est donc caractérisée par une teneur en eau résiduelle libre comprise entre 0 et 800 ppm, préférablement comprise entre 0 et 600 ppm, plus préférablement comprise entre 0 et 400 ppm. De même, la teneur en complexe qui est le réservoir d'eau liée, devra être comprise entre 0 et 3%, préférablement comprise entre 0 et 0,5% et plus préférablement comprise entre 0 et 0,05%.

Dans le cadre de cette invention et en considérant l'existence d'une humidité libre et d'une humidité liée, deux techniques de séchage pourront être envisagées.

La première consiste à traiter le produit issu de l'étape (b) sous sa forme initiale, c'est-à-dire solide. Dans ce cadre , un sécheur conçu pour pouvoir d'une part offrir une capacité de volatilisation importante afin d'éliminer l'eau ou le solvant résiduel, et d'autre part effectuer le séchage dans des conditions suffisamment douces et contrôlées pour éviter toute détérioration thermique du produit, pourra avantageusement être utilisé. En effet, l'opération de séchage est d'autant plus délicate que le gâteau humide contient, contrairement aux autres procédés d'extraction à l'eau, une certaine quantité de méso-LD dont le point de fusion se situe entre 45 et 50°C. Il est donc avantageux d'effectuer cette étape de séchage à une température inférieure à 50°C, ce qui nécessite un travail sous vide ou sous flux gazeux. De plus, l'existence d'un complexe susceptible, sous certaines conditions, de relarguer une quantité d'eau non négligeable ne peut qu'accentuer la nécessité de travailler à faible température afin d'éviter une dégradation accélérée du lactide. Dès lors, tout procédé de séchage et toute technologie connue de l'homme de l'art, favorisant la vaporisation et l'extraction de l'eau ou d'un solvant d'un solide humide, peuvent être envisagés dans le cadre de cette invention, par exemple les mélangeurs sécheurs sous vide ou sous flux gazeux sec, le principe de zéodratation, les sécheurs à plateaux, etc.

La seconde technique préconise une liquéfaction du gâteau humide issu de l'étape (b) et un entraînement de l'eau (libre + liée) par balayage ou barbotage d'un flux gazeux sec. Comme pour la première technique et afin d'assurer la stabilité chimique du lactide, la température de séchage sera de préférence proche de la température minimale requise pour maintenir le lactide humide sous forme liquide. Dès lors, tout procédé et technologie de séchage connue de l'homme de l'art, favorisant l'extraction de l'eau ou d'un solvant, d'un liquide humide peuvent être envisagés dans le cadre de cette invention, comme par exemple : une colonne de lavage de gaz (striping), un sécheur couche mince, des tamis moléculaires, etc.

Par contre, si lors de l'étape de lavage du gâteau par solvant, la teneur en eau résiduelle est amenée par simple extraction à une valeur compatible avec la recristallisation en milieu fondu décrite ci-dessous et si la quantité résiduelle de solvant ne crée pas un problème de compatibilité chimique (ouverture du lactide par le solvant) et technique pour l'étape ultime (pas de diminution de la teneur en solvant durant le procédé), cette étape de séchage pourrait avantageusement être évitée.

D'autre part, si lors du lavage du gâteau, un solvant formant un azéotrope avec l'eau est utilisé, il sera plus aisé et énergétiquement plus intéressant d'en extraire les dernières traces, de façon à atteindre des teneurs résiduelles en eau et éventuellement en solvant suffisamment basses pour permettre une exploitation rentable de l'ultime étape de purification.

### (d) recristallisation en milieu fondu.

Le lactide impur séché obtenu en (c), de composition semblable au produit issu de l'étape (b) à l'exception de la teneur plus faible en eau libre et liée, subira une ultime purification par un procédé de recristallisation en milieu fondu (un ou plusieurs étages), ce qui permettra d'obtenir un lactide d'une pureté chimique et stéréospécifique suffisante pour pouvoir être utilisé comme monomère pour la synthèse du PLA par ouverture de cycle. On entend par pureté suffisante, une teneur en un lactide comprise entre 99,0 et 99,9 % et préférablement encore comprise entre 99,5 et 99,9 %, une teneur en méso-LD comprise entre 0 et 0,5 % et préférablement comprise entre 0 et 0,2 %, une teneur en eau comprise entre 0 et 100 ppm et de préférence comprise entre 0 et 50 ppm, ainsi qu'une acidité comprise entre 0 et 10 méq/kg et de préférence comprise entre 0 et 1 méq/kg.

Le lactide impur séché obtenu en (c) est fondu et subit un refroidissement contrôlé qui va initier la cristallisation. Durant cette étape, les impuretés sont concentrées dans la phase liquide entourant les cristaux. Une fois la cristallisation terminée, la phase liquide est extraite du cristallisoir par gravité, ce qui laisse des cristaux enrobés d'un film chargé d'impuretés. Afin d'éliminer ce film, une refonte partielle des cristaux est opérée. Le liquide ainsi obtenu entraîne le film et est évacué par gravité hors du cristallisoir. L'opération peut être répétée à plusieurs reprises afin d'atteindre la pureté requise. Cette succession d'étapes peut être réalisée suivant un procédé statique ou dynamique.

Finalement, lorsque la pureté souhaitée est atteinte, le contenu du cristallisoir est fondu et récupéré.

Il est important de noter dans le cadre de cette invention que cette étape de purification ultime, ne s'est avérée quantitativement, économiquement et énergétiquement exploitable que grâce au traitement préalable du brut de lactide comme relaté dans les 3 étapes ci-dessus. En effet, il est souhaitable que le produit arrivant à l'étape de recristallisation en milieu fondu soit d'une pureté suffisante (supérieure à 90 % et préférablement encore supérieure à 95 %) pour permettre une exploitation industrielle et économiquement viable du procédé. L'introduction d'un lactide d'une pureté insuffisante accroît considérablement le nombre d'opérations et donc les investissements nécessaires à l'obtention d'un produit final de qualité.

D'autre part, une teneur en eau libre suffisamment faible (<800 ppm et préférablement <400 ppm) est avantageuse pour éviter une détérioration chimique rapide du lactide et donc une réduction importante de la productivité et du rendement du procédé. En effet, l'eau se concentre dans la phase liquide de la première étape du procédé et, étant donné les cycles de chauffe inhérents à la technologie, provoque une ouverture prématurée du lactide. L'efficacité et la rentabilité du procédé étant basées sur un subtil recyclage des différentes fractions, la perte engendrée influe alors directement et de façon très importante sur le rendement final. Il est important de noter que la teneur en complexe dans le produit arrivant à cette étape de cristallisation en milieu fondu devra être drastiquement réduite. En effet, les conditions rencontrées lors de cette étape du procédé pourraient provoquer un relargage de l'eau provenant du complexe et engendreraient le même problème que ci-dessus.

Par le choix judicieux des paramètres régissant la recristallisation en milieu fondu, il sera également possible d'envisager une récupération importante du méso-LD grâce aux faibles pertes à l'étape (a). Ce dernier peut être utilisé pour la synthèse de PLA nécessitant un fin contrôle de la cinétique de dégradation.

Une autre approche de la présente étape de l'invention considère que la viscosité des impuretés du produit à purifier influence fortement le coefficient de transfert de masse au cours de la cristallisation et donc directement la forme des cristaux, la vitesse de cristallisation et le rendement. Dès lors l'ajout, au produit de départ de l'étape (d), d'un solvant permettrait de diminuer la viscosité et donc de résoudre ces problèmes. Le solvant pourrait dans le cadre de cette invention, être mélangé au produit séché issu de l'étape (c) lors d'une purification sans solvant ou être le résidu de solvant introduit lors de l'étape (b) du présent procédé. Cette teneur peut être différente suivant qu'une étape de séchage (c) a été réalisée ou pas.

Dans le cadre de cette invention, ce solvant devra être présent dans des concentrations telles que le principe même de la technologie soit toujours applicable et que l'exploitation industrielle du procédé soit toujours réalisable, à savoir comprises entre 0 et 30 %, préférablement comprises entre 0 et 20 % et plus préférablement comprises entre 0 et 10 %. En effet, l'ajout d'une quantité trop importante de solvant reviendrait à effectuer une recristallisation en solution, nécessiterait l'utilisation de cristallisoirs de capacité plus importante et engendrerait des coûts d'exploitation plus élevés, annulant le bénéfice engendré par l'usage d'un solvant. De plus, celui-ci devra être sélectionné de façon à être inerte vis-à-vis du lactide et à pouvoir être aisément recyclé dans le procédé global de production de PLA au départ de divers substrats carbonés. Parmi les solvants utilisables pour cette étape, on a par exemple les esters d'acide lactique ou l'un des solvants signalés à l'étape (b) .

D'autres détails et particularités de l'invention donnés ci-après de façon non limitative à titre d'exemple, décrivent des formes possibles de réalisation.

### Exemples

### Exemple 1

Un échantillon (feed) de brut de lactide (0,696 kg) contenant 83% de L-LD, 8 % de méso-LD, 1,6% de complexe L-LD hydraté (complexe) et une acidité résiduelle de 570 méq/kg est introduit dans un cristallisoir constitué par un tube vertical en inox de 1 m de long et de 30 mm de diamètre. Ce tube est équipé d'une double enveloppe alimentée en fluide caloporteur par un groupe de chauffe thermostatisé afin de permettre un contrôle de température lors des phases de cristallisation, suage ou refonte du produit. Ce brut est fondu à 105°C et échantillonné pour analyse.

Ensuite, la cristallisation est initiée sur la paroi du cristallisoir par à une diminution progressive de la température au sein de cette paroi, contrôlée par l'évolution de la température du fluide caloporteur présent dans la double enveloppe. Afin d'éviter la formation d'occlusions et d'inclusions au sein des cristaux purs, cette descente en température est soigneusement contrôlée, à une vitesse de l'ordre de 2 à 5°C/h. Durant cette étape, une partie du brut est cristallisé sur les parois du tube, alors que la partie centrale renferme la phase liquide (drain) contenant la majorité des impuretés.

Une fois l'étape de cristallisation terminée (température du fluide caloporteur 60°C), la phase liquide est extraite par gravité et analysée.

A l'issue de cette étape de drainage, les cristaux sont recouverts d'un film constitué d'impuretés que l'étape de suage doit éliminer. Pour ce faire, la surface du tube va être très progressivement chauffée (de 60 à 98°C) de façon à faire fondre la surface des cristaux caractérisée par une moindre pureté, donc par un point de fusion inférieur à celui du produit pur. Suivant la nature du brut, la fraction récoltée par gravité durant le suage représente de 5 à 25% de la charge initiale.

Lors de la dernière étape, le cristallisoir est amené (à 4°C/min) à la température de fusion du produit (97-102°C) afin de liquéfier l'ensemble de la charge (melt) qui sera récoltée par gravité et ensuite analysée.

Afin d'obtenir un produit final répondant aux spécifications minimales requises pour un lactide utilisé dans la synthèse du PLA, le produit obtenu subira un deuxième, voire un troisième étage de purification en utilisant une procédure identique à celle exposée ci-dessus.

Le tableau I démontre l'enrichissement des fractions intermédiaires en impuretés ainsi que l'accroissement du rendement massique des fractions récoltées en fonction de la composition des produits à purifier.

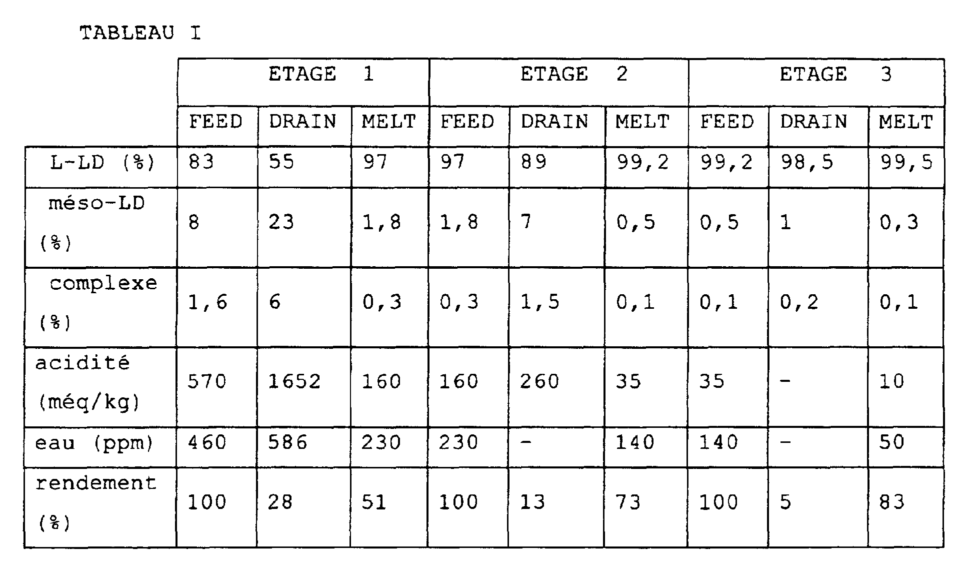

Les teneurs en L-LD, méso-LD et complexe sont déterminées par GC après estérification des composés carboxylés. Les acidités sont mesurées par titration au methoxyde de sodium dans un solvant anhydre avec de la phénolphtaléine comme indicateur. Les teneurs en eau ont été déterminées suivant Karl Fisher.

### Exemple 2

Un échantillon de brut de lactide contenant 77,2% de L-LD, 8,6 % de méso-LD, 1,2% de complexe et une acidité résiduelle de 1.840 méq/kg va subir un traitement de prépurification, avant une purification par recristallisation en milieu fondu comme énoncé à l'exemple 1.

Aux 2.583 kg de brut maintenu à 90°C sont ajoutés 25% en poids d'eau froide. Le mélange est rapidement amené à sa température de cristallisation, où il y est maintenu durant 30 min afin de favoriser la nucléation des cristaux. Ensuite, la température est progressivement diminuée de façon à atteindre une température finale de 25°C.

Le mélange est ensuite essoré à une vitesse de 1.500 tours/min et 1.553 kg de produit constitué de gros cristaux blancs sont récoltés. L'analyse de ce produit avant et après séchage, donne les résultats repris au tableau II.

| TABLEAU II | | |
|---|---|---|
| | Avant séchage | Après séchage |
| L-LD (%) | 94,3 | 85,8 |
| méso-LD (%) | 0,7 | 0,7 |
| complexe (%) | 3,5 | 11,8 |
| eau (ppm) | 5000 | 440 |

La teneur en eau étant beaucoup trop importante pour pouvoir traiter directement le produit par recristallisation en milieu fondu, on fait barboter un flux d'azote sec durant 1,5 h au sein du produit maintenu à 110°C. Ce traitement permet de réduire la teneur en eau à 440 ppm mais augmente la concentration en complexe au détriment du L-LD.

Le produit séché issu de ce traitement subira deux ou trois étages de purification par recristallisation en milieu fondu, en utilisant une procédure identique à celle exposée dans l'exemple 1.

Le tableau III montre un accroissement de l'efficacité de la purification en milieu fondu. En effet, au départ d'un feed de moindre pureté, deux étages suffisent pour atteindre la qualité requise. D'autre part, on peut remarquer que la présence de ce complexe influe fortement et de façon négative le rendement massique des fractions récoltées durant cette étape ultime de purification.

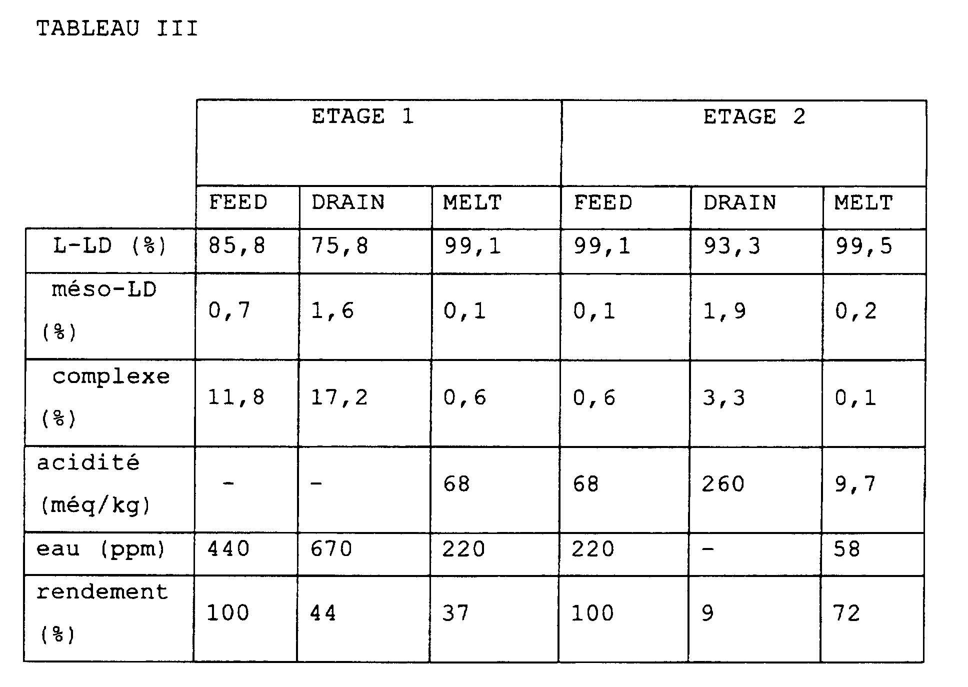

### Exemple 3

Un échantillon de brut de lactide contenant 84,9% de L-LD, 5,5 % de méso-LD, 3,3% de complexe et une acidité résiduelle de 830 méq/kg, va subir un traitement similaire à celui de l'exemple 2, mis à part les phases d'essorage et de séchage qui seront adaptées afin de réduire au minimum la formation du complexe.

Aux 2.587 kg de brut maintenu à 90°C sont ajoutés 25% en poids d'eau froide. Le mélange est rapidement amené à sa température de cristallisation, où il y est maintenu durant 30 min. Ensuite la température est diminuée de façon à atteindre une température finale de 25°C.

Le mélange est ensuite essoré à 2.000 tours/min et 1.786 kg de produit constitué de gros cristaux blancs sont récoltés. Ces cristaux sont séchés sous vide à 45°C, ce qui permet d'extraire l'eau libre mais également l'eau liée sous forme de complexe. En effet, la disparition du complexe correspond à une augmentation de la teneur en L-LD. Le produit séché obtenu est caractérisé par le tableau IV.

| TABLEAU IV | | |
|---|---|---|
| | Avant séchage | Après séchage |
| L-LD (%) | 90,8 | 97,6 |
| méso-LD (%) | 0,9 | 0,7 |
| complexe (%) | 5 | 1,1 |
| eau (ppm) | 3450 | 370 |

Le produit séché issu de ce traitement sera soumis à des recristallisations en milieu fondu en utilisant une procédure identique à celle exposée dans l'exemple 1.

Le tableau V démontre par comparaison aux résultats obtenus à l'exemple 2, un accroissement du rendement massique des fractions récoltées dans ce procédé de purification lorsque la teneur en complexe dans le produit de départ est plus faible.

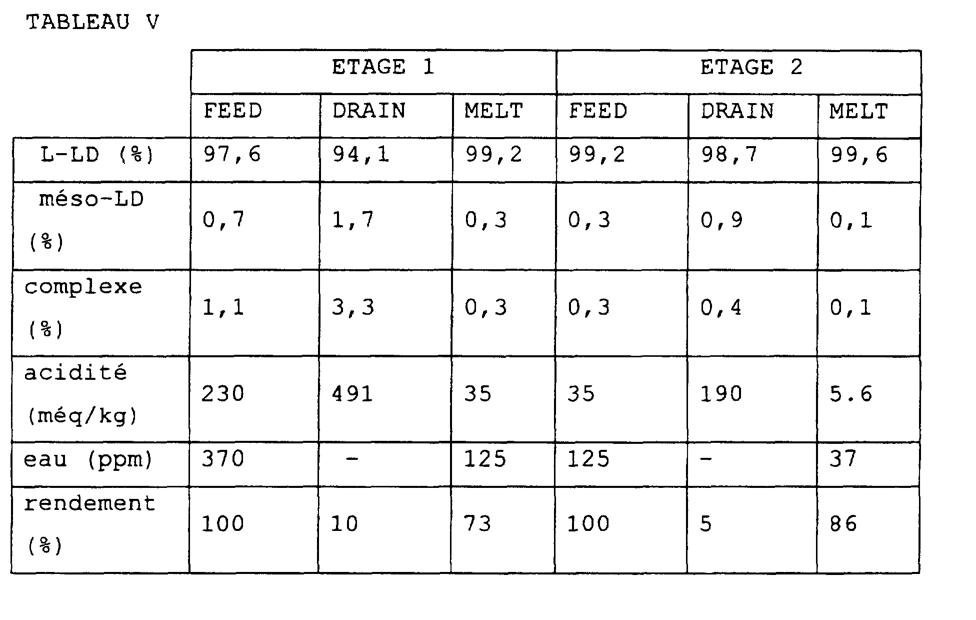

### Exemple 4

Cet exemple démontre l'efficacité de la méthode de purification pour des mélanges pauvres en lactide, sous-produits du procédé de purification. Un échantillon de brut de lactide contenant 41,9% de L-LD, 14,3 % de méso-LD et 2,2% de complexe, va subir un traitement similaire à celui de l'exemple 3.

Aux 1,082 kg de brut maintenu à 80°C sont ajoutés 25% en poids d'eau froide. Le mélange est rapidement amené à sa température de cristallisation, où il y est maintenu durant 30 min. Ensuite, la température est diminuée de façon à atteindre une température finale de 25°C.

Le mélange est ensuite essoré et 0,400 kg de produit constitué de gros cristaux blancs sont récoltés et séchés. Le produit séché obtenu est caractérisé par le tableau VI.

| TABLEAU VI | | |
|---|---|---|
| | Avant séchage | Après séchage |
| L-LD (%) | 91,1 | 93,2 |
| méso-LD (%) | 2,6 | 2,2 |
| complexe (%) | 2,4 | 0,8 |
| eau (ppm) | 4200 | 800 |

Le produit séché issu de ce traitement peut être traité par recristallisation en milieu fondu en utilisant une procédure identique à celle exposée dans l'exemple 1. Par rapport aux procédés conventionnels de production de PLA où des recyclages sont mis en place, l'avantage de cette technique réside dans la possibilité offerte de recycler le lactide en tant que tel et non plus sous forme de lactate.

## Revendications

1. Procédé de purification de l'ester cyclique dimérique d'acide lactique au départ d'un brut de lactide comportant des impuretés, le procédé comprenant les étapes suivantes :
a) cristallisation extractive et contrôlée du brut de lactide, en milieu aqueux, en contrôlant la géométrie des cristaux formés et en provoquant une ségrégation de phases entre le lactide (phase solide) et les impuretés (phase liquide), favorisant une extraction aqueuse des impuretés;
b) séparation de la suspension de cristaux obtenue en (a), en une phase liquide pauvre en lactide et chargée des impuretés et en un gâteau humide riche en cristaux de lactide;
c) séchage du gâteau humide obtenu en (b);
d) recristallisation en milieu fondu du lactide impur séché obtenu en (c) et récupération du lactide purifié.

2. Procédé suivant la revendication 1, **caractérisé en ce que** le brut de lactide comprend un mélange d'acide lactique et/ou d'ester d'acide lactique et leurs oligomères respectifs, d'eau et/ou d'alcool, ainsi que les différentes formes diastéréoisomères du lactide ou leurs mélanges.

3. Procédé suivant la revendication 2, **caractérisé en ce que** le brut de lactide est obtenu par mélange de fractions issues de procédés de synthèse ou de purification de lactide.

4. Procédé suivant l'une quelconque des revendications précédentes, **caractérisé en ce que** le brut de lactide présente une teneur en un diastéréoisomère du lactide comprise entre 30 et 90 % et préférablement comprise entre 40 et 85 %, une teneur en eau comprise entre 0 et 2 % et préférablement comprise entre 0 et 1 %, une teneur en acide lactique et oligomères d'acide lactique comprise entre 0 et 50 %, et une teneur en méso-lactide et en l'autre diastéréoisomère du lactide comprise entre 0 et 30 %.

5. Procédé suivant l'une quelconque des revendications précédentes, **caractérisé en ce que** la cristallisation extractive et contrôlée (a) comprend une première phase de germination progressive du lactide, et une seconde phase de croissance des cristaux avec rejet des impuretés en phase aqueuse.

6. Procédé suivant la revendication 5, **caractérisé en ce que** la phase de germination progressive est initiée par le maintien du mélange à une température légèrement inférieure à celle de cristallisation du lactide dans le mélange.

7. Procédé suivant la revendication 5, **caractérisé en ce que** la phase de croissance avec extraction des impuretés est assurée par une diminution contrôlée de la température du mélange, favorisant la croissance de cristaux de lactide.

8. Procédé suivant l'une quelconque des revendications précédentes, **caractérisé en ce que** la cristallisation extractive et contrôlée (a) est réalisée à une température comprise entre 100 et 0°C et préférablement comprise entre 80 et 10°C.

9. Procédé suivant l'une quelconque des revendications précédentes, **caractérisé en ce que** l'étape de cristallisation extractive et contrôlée (a) est effectuée sur un mélange dont la teneur en eau ajoutée par rapport au brut de lactide est comprise entre 0 et 40 %, de préférence comprise entre 0 et 30 %.

10. Procédé suivant l'une quelconque des revendications précédentes, **caractérisé en ce que** l'étape de cristallisation extractive et contrôlée (a) comprend l'utilisation d'un réacteur avec agitation, disposant d'une capacité de thermostatisation, et d'un système d'extraction également adapté aux produits pâteux, avec un temps de séjour compris entre 1 et 90 min, de préférence compris entre 1 et 60 min.

11. Composition de la suspension de cristaux obtenue après l'étape (a) **caractérisée en ce qu'**elle comporte notamment une teneur en eau comprise entre 1 et 40 % et préférablement comprise entre 1 et 25 %, une teneur en un diastéréoisomère du lactide comprise entre 35 et 90 %, préférablement comprise entre 40 et 90 %, une teneur en acide lactique et oligomères d'acide lactique comprise entre 0 et 10 % et préférablement comprise entre 0 et 5 %, et une teneur en méso-lactide et en l'autre diastéréoisomère du lactide.

12. Composition suivant la revendication 11, **caractérisée en ce que** le diastéréoisomère du lactide comprend le lactide proprement dit et un complexe de lactide, qui consiste en une molécule de lactide liée de façon réversible, par pont d'hydrogène, à une molécule d'eau.

13. Procédé suivant l'une quelconque des revendications de procédé précédentes, **caractérisé en ce que** l'étape (b) de séparation est une séparation par centrifugation ou autre, qui permet d'atteindre une teneur résiduelle en eau libre dans le gâteau humide comprise entre 0 et 3 %, préférablement comprise entre 0 et 1 %.

14. Procédé suivant la revendication 13, **caractérisé en ce que** l'étape de séchage (c) est remplacée par un lavage centrifuge par solvant du gâteau humide riche en cristaux de lactide obtenu à l'étape (b), pour en extraire l'eau.

15. Composition du gâteau humide obtenu après l'étape (b) **caractérisée en ce qu'**elle comporte notamment une teneur en eau libre comprise entre 0 et 5 % et préférablement comprise entre 0 et 2 %, une teneur totale en lactide comprise entre 75 et 98 %, préférablement comprise entre 85 et 98 %, une teneur en acide lactique et oligomères d'acide lactique comprise entre 0 et 5 %, préférablement comprise entre 0 et 3 %, et une teneur en méso-lactide et en l'autre diastéréoisomère du lactide.

16. Composition suivant la revendication 15, **caractérisée en ce que** la teneur totale en lactide comprenant la teneur en lactide diastéréoisomère souhaité ainsi que la teneur en lactide complexé, est comprise entre 1 et 30 % et préférablement comprise entre 1 et 20 %.

17. Procédé suivant l'une quelconque des revendications de procédé précédentes, **caractérisé en ce que** l'étape (c) comprend un séchage qui atteint une teneur résiduelle en eau libre dans le lactide impur séché comprise entre 0 et 800 ppm, préférablement comprise entre 0 et 400 ppm, et une teneur résiduelle en eau liée sous forme de lactide complexe comprise entre 0 et 3 %, préférablement comprise entre 0 et 0,5 %.

18. Procédé suivant l'une quelconque des revendications de procédé précédentes, **caractérisé en ce que** le séchage (c) en phase solide du gâteau humide issu de (b) est réalisé sous vide ou sous flux gazeux sec, à une température inférieure à 50°C.

19. Procédé suivant l'une quelconque des revendications de procédé précédentes, **caractérisé en ce que** l'étape de séchage (c) en phase liquide comprend la liquéfaction préalable du gâteau humide issu de (b) et un entraînement de l'eau (libre et liée) par un barbotage et/ou balayage d'un flux gazeux sec dans la solution.

20. Procédé suivant la revendication 19, **caractérisé en ce que** la température de séchage (c) légèrement supérieure à la température de liquéfaction du lactide humide, est comprise entre 90 et 130°C et préférablement comprise entre 95 et 115°C.

21. Procédé suivant la revendication 18 ou 19, **caractérisée en ce que** le flux gazeux sec est un gaz inerte ou de l'air et en ce que ce flux gazeux est éventuellement préchauffé.

22. Procédé suivant l'une quelconque des revendications de procédé précédentes, **caractérisé en ce que** l'étape (d) comporte une ou plusieurs recristallisations en milieu fondu du lactide impur séché obtenu en (c).

23. Procédé suivant la revendication 22, **caractérisé en ce qu'**un réducteur de viscosité est mélangé au lactide impur séché issu de l'étape (c) afin d'augmenter la vitesse de cristallisation, le coefficient de transfert de masse et l'efficacité de la recristallisation en milieu fondu.

24. Procédé suivant la revendication 23, **caractérisé en ce que** le réducteur de viscosité est un solvant introduit à l'issue de l'étape de séchage (c), et choisi parmi les cétones, les éthers, les solvants aromatiques ou aliphatiques, les solvants à base de silicone, les solvants halogénés, les alcools et les esters d'acide lactique.

25. Procédé suivant la revendication 23, **caractérisé en ce que** le réducteur de viscosité est le solvant résiduel introduit lors de l'étape de séparation centrifuge (b) du procédé, et choisi parmi les cétones, les éthers, les solvants aromatiques ou aliphatiques, les solvants à base de silicone, les solvants halogénés, les alcools et les esters d'acide lactique.

26. Procédé suivant l'une quelconque des revendications 23 à 25, **caractérisé en ce que** la teneur en réducteur de viscosité est comprise entre 0 et 30 %, préférablement comprise entre 0 et 20 %.

27. Procédé suivant l'une quelconque des revendications de procédé précédentes, **caractérisé en ce que** la teneur en lactide à l'issue de l'étape (d) est comprise entre 99,0 et 99,9 %, préférablement comprise entre 99,5 et 99,9 %, la teneur en méso-lactide est comprise entre 0 et 0,5 %, préférablement comprise entre 0 et 0,2 %, la teneur en eau est comprise entre 0 et 100 ppm, préférablement comprise entre 0 et 50 ppm, la teneur acide lactique et oligomères est comprise entre 0 et 10 méq/kg, préférablement comprise entre 0 et 1 méq/kg.

28. Procédé de purification de l'ester cyclique dimérique d'acide glycolique au départ d'un brut de glycolide comportant des impuretés, le procédé comprenant les étapes suivantes :
a) cristallisation extractive et contrôlée du brut de glycolide, en milieu aqueux, en contrôlant la géométrie des cristaux formés et en provoquant une ségrégation de phases entre le glycolide (phase solide) et les impuretés (phase liquide), favorisant une extraction aqueuse des impuretés;
b) séparation de la suspension de cristaux obtenue en (a), en une phase liquide pauvre en glycolide et chargée des impuretés et en un gâteau humide riche en cristaux de glycolide;
c) séchage du gâteau humide obtenu en (b);
d) recristallisation en milieu fondu du glycolide impur séché obtenu en (c) et récupération du glycolide purifié.
